(19) Europäisches Patentamt
European Patent Office
Office européen des brevets

(11) Publication number: **0 079 740**
**B1**

(12) # EUROPEAN PATENT SPECIFICATION

(45) Date of publication of patent specification: **13.11.85**

(51) Int. Cl.[4]: **C 07 D 401/12,** A 61 K 31/47

(21) Application number: **82305928.2**

(22) Date of filing: **08.11.82**

(54) **Anthraniloyloxyalkanoates.**

(30) Priority: **14.11.81 GB 8134398**

(43) Date of publication of application:
**25.05.83 Bulletin 83/21**

(45) Publication of the grant of the patent:
**13.11.85 Bulletin 85/46**

(84) Designated Contracting States:
**AT BE CH FR GB LI SE**

(56) References cited:
**FR-M- 6 195**
**GB-A-2 031 412**

(73) Proprietor: **RECORDATI S.A. CHEMICAL and PHARMACEUTICAL COMPANY**
**Corso S. Gottardo 54**
**CH-6830 Chiasso (CH)**

(72) Inventor: **Nardi, Dante**
**Via T. Gulli 47**
**Milan (IT)**
Inventor: **Motta, Gianni**
**Via Piave 34A**
**Meda Milan (IT)**
Inventor: **Graziani, Gabrielle**
**Via Dei Platani 8**
**Arese Milan (IT)**
Inventor: **Testa, Rodolfo**
**Via Astesani 64**
**Milan (IT)**

(74) Representative: **SERJEANTS**
**25 The Crescent King Street**
**Leicester, LE1 6RX (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 079 740 B1

**Description**

The invention relates to N-substituted-4-(optionally substituted)-2-,3- or 4-piperidyl N-(7- or 8-substituted-4-quinolyl)-anthraniloyloxyalkanoates, to pharmaceutically acceptable salts thereof, to processes for the preparation of these esters, and to pharmaceutical compositions containing them.

FR—M—6195 discloses that alkoxycarbonylmethyl esters of N-[7 (or 8)-chloro-4-quinolyl[-anthranilic acid have antiinflammatory properties and analgesic properties. GB—A—2031412 discloses that 1-methyl-4-piperidyl esters of N-[(7-chloro- or 7- or 8-trifluoromethyl)-4-quinolyl]-anthranilic acid also have such properties. According to the invention, it has been found that certain 1-[N-substituted-2-,3- or 4-piperidyloxycarbonyl]-alkyl esters of these acids also have such properties and, surprisingly, are also strong inhibitors of platelet aggregation. The piperidyl group is not limited to a 4-piperidyl group as in GB—A—2031412; the N-substitution off the piperidyl group is not limited to a methyl group as in GB—A—2031412; the piperidyl group may be 4-phenyl substituted, unlike GB—A—2031412; and the esters are not limited to substituted methyl ester as in FR—M—6195, but embrance 1-substituted alkyl esters.

The invention provides N-substituted-4-(optionally substituted)-2-,3- or 4-piperidyl N-(7- or 8-substituted-4-quinolyl)-anthraniloyloxyalkanoates having the general formula I

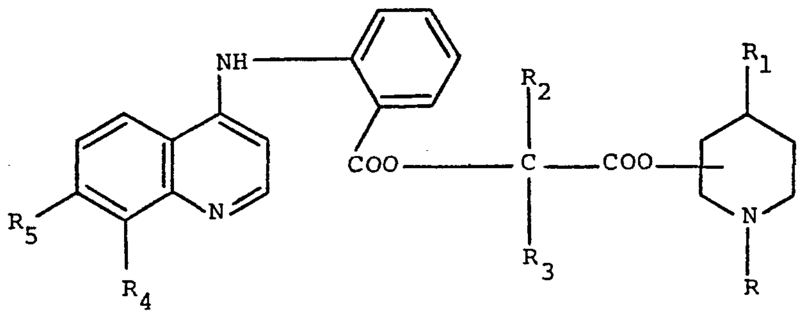

I

in which R represents a straight chain or branched chain alkyl group having from 1 to 4 carbon atoms; an alkenyl or alkynyl group having from 2 to 6 carbon atoms; a benzyl, phenethyl, 4-nitro-phenethyl or 4-aminophenethyl group; or a phenacyl, benzoylethyl, β-hydroxy-phenethyl or α-hydroxy-phenylpropyl group, optionally substituted in the phenyl ring by one or more of a halogen atom, a trifluoromethyl, nitro or amino group, an alkyl group having from 1 to 4 carbon atoms or an alkoxy group having from 1 to 4 carbon atoms; $R_1$ represents a hydrogen atom or a phenyl group, each of $R_2$ and $R_3$ independently represents a hydrogen atom or an alkyl group having from 1 to 4 carbon atoms, one of $R_4$ and $R_5$ represents a chlorine atom or a trifluoromethyl group and the other of $R_4$ and $R_5$ represents a hydrogen atom. The invention further provides pharmaceutically acceptable salts of such esters.

The compounds according to the invention have been found to exhibit powerful analgesic and antiinflammatory properties. They are also strong inhibitors of platelet aggregation and antidepressant agents.

The invention further provides a method for the preparation of the compounds according to the invention, the method comprising reacting a substituted piperidine of the general formula II

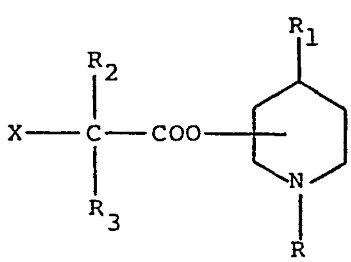

II

wherein X represents a halogen atom and R, $R_1$, $R_2$ and $R_3$ are as hereinbefore defined with an anthranilic acid derivative of the general formula III

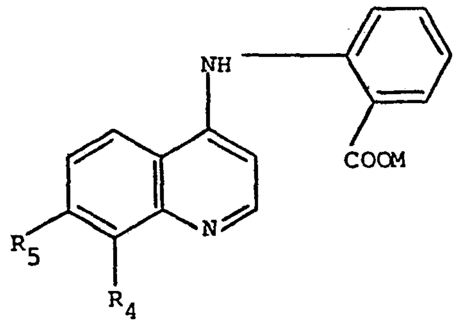

III

wherein $R_4$ and $R_5$ are as hereinbefore defined and M represents a hydrogen atom or an alkali metal atom.

The intermediates II are novel, and may be obtained by reacting a piperidinol of the general formula IV or a hydrochloride thereof

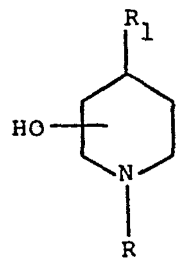

IV

wherein R and $R_1$ are as hereinbefore defined with a haloalkanoyl chloride of the general formula V

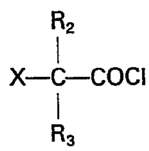

V

wherein $R_2$, $R_3$ and X are as hereinbefore defined. The condensation is preferably carried out in the presence of an inert organic solvent, such as dimethylformamide. The reactants are generally used in equimolar proportions, the temperature ranging from 20° to 50°C. The piperidinols IV are easily obtained from piperidinols of the general formula VI

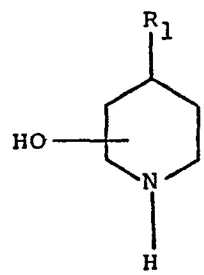

VI

wherein $R_1$ is as hereinbefore defined by attaching in the usual way a suitable substituent R to the nitrogen atom.

The anthranilic acid derivatives III are well known in the art: they can be employed as the free acid or as alkali metal salts. When the free acid is used, the reaction with the piperidine derivative II is carried out in the presence of a solvent, such as dimethylformamide. When an alkali metal salt is employed, the reaction is preferably carried out in dimethylformamide. Both when employing the free acid and the alkali metal salt, an alkali metal carbonate is always added as acid bonding agent, and the temperature may range from 20° to 100°C, preferably from 50°C to 100°C. At the end of the reaction, the ester thus formed may be purified in conventional manners, for example by chromatography and crystallization. The compounds of the invention may be employed as such or transformed into pharmaceutically acceptable acid addition salts. These salts, according to the invention, may be prepared from the free bases according to conventional methods such as addition of an acid to the base dissolved in a suitable solvent.

The invention additionally provides a pharmaceutical composition comprising a compound having the general formula I as above defined or a pharmaceutically acceptable salt thereof in admixture with a pharmaceutically acceptable diluent or carrier.

The active compounds according to the invention exhibit a good analgesic and antiinflammatory action, whereas they show a low toxicity. They are also strong inhibitors of platelet aggregation and antidepressant agents. The $LD_{50}$ values, determined per os in the mouse, are greater than 3000 mg/Kg for many of the tested compounds, the other values being greater than 1000 mg/Kg.

The analgesic activity, determined in the mouse with the writhing test (Sigmund et al., Proc. Soc. Exp. Biol. med., *95*, 729, 1957) and given as $ED_{50}$ mM/Kg, is from 0.06 to 0.1; the inflammatocy activity, determined pn the rat with the carragenina test (Winter et al., Proc. Soc. Exp. Biol. Med., *111*, 544, (1962) and given as above, ranges from 0.08 to 0.4.

The compounds of the invention are highly active as antidepressant agents, and can be used to treat illness which involve increased platelet aggregation, e.g. cerebral thrombosis, cerebral infarction, cardiac infarction and arteriosclerotic disorders.

Example 1

Preparation of intermediates II

11.3 g of Chloroacetylchloride was added to a solution of 11.4 g of N-methyl-3-piperidinol in 20 ml of dimethylformamide at 20 to 25°C. The product, which formed immediately, was allowed to stand at this temperature for 24 hours. It was then collected by filtration and recrystallized from isopropanol to give 12.4 g of N-methyl-3-piperidyl chloroacetate hydrochloride (II, $R=CH_3$, $R_1=R_2=R_3=H$, X=Cl), m.p. 168°C.

Operating as described above, but employing N-methyl-4-piperidinol; N-ethyl-4-piperidinol; N-butyl-4-piperidinol; N-benzyl-4-piperidinol; N-methyl-4-phenyl-4-piperidinol; N-phenethyl-4-piperidinol; and N-propargyl-4-piperidinol in place of N-methyl-3-piperidinol, there were obtained respectively N-methyl-4-piperidyl chloroacetate hydrochloride (II, $R=CH_3$, $R_1=R_2=R_3=H$, X=Cl), m.p. 177—179°C; N-ethyl-4-piperidyl chloroacetate hydrochloride (II, $R=C_2H_5$, $R_1=R_2=R_3=H$, X=Cl), m.p. 176—178°C; N-butyl-4-piperidyl chloroacetate hydrochloride (II, $R=C_4H_9$, $R_1=R_2=R_3=H$, X=Cl), m.p. 205—206°C; N-benzyl-4-piperidyl chloroacetate hydrochloride (II, $R=C_6H_5CH_2$, $R_1=R_2=R_3=H$ X=Cl), m.p. 214—215°C; N-methyl-4-phenyl-4-piperidyl chloroacetate hydrochloride (II, $R=CH_3$, $R_1=C_6H_5$, $R_2=R_3=H$, X=Cl), m.p. 181—183°C; N-phenethyl-4-piperidyl chloroacetate hydrochloride (II, $R=C_6H_5CH_2CH_2$, $R_1=R_2=R_3=H$, X=Cl), m.p. 211—213°C; and N-propargyl-4-piperidyl chloroacetate hydrochloride (II, R=CH C—$CH_2$, $R_1=R_2=R_3=H$, X=Cl), m.p. 153—154°C.

Example 2

N-Methyl-3-piperidyl N-(7-chloro-4-guinolyl)anthraniloyloxyacetate (I, $R=CH_3$, $R_1=R_2=R_3=R_4=H$, $R_5=Cl$)

4.56 g of N-methyl-3-piperidyl chloroacetate hydrochloride prepared as described in Example 1, 6.4 g of the sodium salt of N-(7-chloro-4-quinolyl)-anthranilic acid and 2.8 g of anhydrous potassium carbonate were heated together under stirring in 40 ml of dimethylformamide at 80°C for 5 hours. The solution was then cooled and filtered. The solvent was evaporated off in vacuo at 50—70°C. The oily residue thus obtained was treated with water and sodium carbonate, and then extracted with ethyl acetate. The organic phase was washed with water and dried on anhydrous sodium sulphate. The ethyl acetate was evaporated off, and the product was purified by chromatography on an alumina column using chloroform as eluant. The fractions were pooled and concentrated to give 2.5 g of the title product, m.p. 135—137°C.

Example 3

N-Methyl-4-piperidyl N-(7-chloro-4-quinolyl)-anthraniloyloxyacetate (I, $R=CH_3$, $R_1=R_2=R_3=R_4=H$, $R_5=Cl$)

Operating as described in Example 2, but employing N-methyl-4-piperidyl chloroacetate hydrochloride, prepared as described in Example 1, in place of N-methyl-3-piperidyl chloroacetate hydrochloride, there was obtained the title compound, m.p. 150—151°C.

Example 4

N-Methyl-4-phenyl-4-piperidyl N-(7-chloro-4-quinolyl)-anthraniloyloxyacetate (I, $R=Ch_3$, $R_1=C_6H_5$, $R_2=R_3=R_4=H$, $R_5=Cl$)

15.2 g of N-methyl-4-phenyl-4-piperidyl chloroacetate hydrochloride, prepared as described in Example 1, 16 g of the sodium salt of N-(7-chloro-4-quinolyl)-anthranilic acid and 7g of anhydrous potassium carbonate were heated together in 100 ml of dimethylformamide, following the procedure described in Example 2. 10.5 g of the title compound were obtained and were recrystallized from methanol. m.p. 148—149°C.

Example 5

N-Ethyl-4-piperidyl N-(7-chloro-4-quinolyl)-anthraniloyloxyacetate (I, $R=C_2H_5$, $R_1=R_2=R_3=R_4=H$, $R_5=Cl$)

Operating as described in Example 2, but using N-ethyl-4-piperidyl chloroacetate hydrochloride, prepared as described in Example 1, in place of N-methyl-3-piperidyl chloroacetate hydrochloride, there was obtained the title compound, m.p. 132—134°C.

Example 6

N-Butyl-4-piperidyl N-(7-chloro-4-quinolyl)-anthraniloyloxyacetate (I, $R=C_4H_9$, $R_1=R_2=R_3=R_4=H$, $R_5=Cl$)

Operating as described in Example 2, but using N-butyl-4-piperidyl chloroacetate hydrochloride,

prepared as described in Example 1, in place of N-methyl-3-piperidyl chloroacetate hydrochloride, there was obtained the title compound, m.p. 111—112°C.

Example 7

N-Methyl-4-piperidyl N-(7-trifluoromethyl-4-quinolyl)-anthraniloyloxyacetate (I, $R=CH_3$, $R_1=R_2=R_3=R_4=H$, $R_5=CF_3$)

A mixture of 9.12 g of N-methyl-4-piperidyl chloroacetate hydrochloride, prepared as described in Example 1, 14.5 g of the sodium salt of N-(7-trifluoromethyl-4-quinolyl)-anthranilic acid and 5.6 g of anhydrous potassium carbonate was heated at 80°C for 5 hours in 80 ml of dimethylformamide. The mixture was cooled, filtered, and evaporated to dryness in vacuo. Water was added to the oily residue obtained, and the mixture was extracted with ethyl acetate. The extract was dried on anhydrous sodium sulphate, and the solvent was evaporated off. The residue was washed with diethyl ether. The etheric phase was purified chromatographically on an alumina column using chloroform as eluant. The title compound, m.p. 101—102°C, was recrystallised from hexane.

Example 8

N-Benzyl-4-piperidyl N-(7-chloro-4-quinolyl)-anthraniloyloxyacetate (I, $R=C_6H_5CH_2$, $R_1=R_2=R_3=R_4=H$, $R_5=Cl$)

Operating as described in Example 7, but commencing by heating 12.16 g of N-benzyl-4-piperidyl chloroacetate hydrochloride, prepared as described in Example 1, 12.8 g of the sodium salt of N-(7-chloro-4-quinolyl)-anthranilic acid and 5.6 g of anhydrous potassium carbonate in 80 ml of dimethylformamide, there was obtained 9.8 g of the title compound, m.p. 125—126°C.

Example 9

N-Phenethyl-4-piperidyl N-(7-chloro-4-quinolyl)-anthraniloyloxyacetate (I, $R=C_6H_5CH_2CH_2$, $R_1=R_2=R_3=R_4=H$, $R_5=Cl$)

The title compound, m.p. 118—120°C, was prepared (yield 6 g) from 15.9 g of N-phenethyl-4-piperidyl chloroacetate hydrochloride, prepared as described in Example 1, and 16 g of the sodium salt of N-(7-chloro-4-quinolyl)-anthranilic acid, following the method described in Example 7.

Example 10

N-Phenacyl-4-piperidyl N-(7-chloro-4-quinolyl)-anthraniloyloxyacetate (I, $R=C_6H_5COCH_2$, $R_1=R_2=R_3=R_4=H$, $R_5=Cl$)

A mixture of 20 g of N-phenacyl-4-piperidyl chloroacetate hydrochloride, 19.2 g of the sodium salt of N-(7-chloro-4-quinoyl)-anthranilic acid, 8.4 g of potassium carbonate and 120 ml of dimethylformamide, was heated at 80°C for 6 hours. When heating was over, the inorganic salts were filtered off and the solution evaporated to dryness in vacuo. The residue thus obtained was treated with water and the solid collected by filtration was purified on a silica gel column using ethyl acetate as eluent. Intermediate fractions containing the title compound were evaporated to dryness and the solid thus obtained was crystallized from acetone. Mp 154—155°C. Yield 9 g (27%).

Example 11

N-(*p*-nitro-phenethyl)-4-piperidyl N-(7-chloro-4-quinolyl)-anthraniloyloxyacetate (I, R=4-nitrophenethyl, $R_5=Cl$, $R_1=R_2=R_3=R_4=H$).

A mixture of 18.1 g of N-(*p*-nitro-phenethyl)-4-piperidyl chloroacetate hydrochloride, 16 g of the sodium salt of N-(7-chloro-4-quinolyl)-anthranilic acid and 7 g of potassium carbonate in 100 ml of dimethylformamide was refluxed for 5 hours at 80°C. At the end of the reaction the insoluble inorganic salts were filtered off and the mixture was evaporated to dryness at 50—70°C. The oily residue was treated with water and the solid, collected by filtration, was purified on a silica gel column using ethyl acetate as eluent. The fractions containing the pure title compound were gathered, the solvent was evaporated off in vacuo and the residue crystallized from ethyl acetate. Mp 135—136°C. Yield 19.5 g (66%).

0.2 g of 5% palladium-on-carbon was added to 5.9 g of this product in 100 ml of acetic acid, and the whole was hydrogenated. When the absorption of hydrogen was over the catalyst was filtered off, the solution was diluted with 150 ml of water, a 20% solution of sodium carbonate was added until pH 8 and the solution was extracted with ethyl acetate. The solvent was then evaporated off and the residue chromatographed on a silica gel column using acetone as eluent. The first fractions were collected, the solvent was evaporated off and the residue purified by conversion into its hydrochloride.

NMR ($CDCl_3$)

δ 8.6 1H (d) quinoline
4.9 1H (m) piperidine
4.85 2H (s) $-O-CH_2-COO$
3.35 2H (bs) $-NH_2$

Mp: 214—216°C (with decomposition). In formula I, R=4-amino-phenethyl.

Example 12

N-allyl-4-piperidyl N-(7-chloro-4-quinolyl)-anthraniloyloxyacetate (I, R=$CH_2$—CH=$CH_2$, $R_5$=Cl, $R_1$=$R_2$=$R_3$=$R_4$=H).

A mixture of 12.7 g of N-allyl-4-piperidyl chloroacetate hydrochloride, 16 g of the sodium salt of N-(7-chloro-4-quinolyl)-anthranilic acid and 7 g of anhydrous potassium carbonate in 100 ml of dimethylformamide was stirred and heated at 80°C for 5 hours. At the end of the reaction the insoluble inorganic salts were filtered off and the mixture was evaporated to dryness in vacuo at 50—70°C. The oily residue was treated with water and the solid, collected by filtration, was purified on a silica gel column using ethyl acetate as eluent. The fractions containing the title compound were gathered, the solvent evaporated off in vacuo and the residue crystallized from ethanol. Mp 115—117°C; yield 7.4 g (31%).

Using N-propargyl-4-piperidyl chloroacetate instead of N-allyl-4-piperidyl chloroacetate, the corresponding ester was obtained. Crystallization from ethyl acetate, mp 111—113°C, yield 10.5 g (44%), employing same amounts of starting materials (I, R=$CH_2$—C≡CH).

Example 13

N-benzyl-4-piperidyl N(8-trifluoromethyl-4-quinolyl)-anthraniloyloxyacetate (I, R=benzyl, $R_1$=$R_2$=$R_3$=$R_5$H, $R_4$=$CF_3$).

Operating as described in Example 12, but starting from 17.7 g of N-benzyl-4-piperidyl chloroacetate hydrochloride, 15.2 g of the sodium salt of the corresponding anthranilic acid and 7 g of potassium carbonate, 17 g of the title compound were obtained. Mp 99—100°C, yield 60%.

Example 14

N-methyl-4-phenyl-4-piperidyl N-(8-trifluoromethyl-4-quinolyl)-anthraniloyloxyacetate (I, R=$CH_3$, $R_1$=Ph, $R_2$=$R_3$=$R_5$=H, $R_4$=$CF_3$).

A mixture of 1.52 g of N-methyl-4-phenyl-4-piperidyl chloroacetate hydrochloride, 1.77 g of the sodium salt of N-(8-trifluoromethyl-4-quinolyl)-anthranilic acid and 0.7 g of potassium carbonate in 10 ml of dimethylformamide was stirred for 48 hours at 20—25°C. At the end of the reaction the inorganic salts were filtered off. The solution was evaporated to dryness and treated with water, and the solid sollected by filtration. This solid was then refluxed with hexane, filtered and recrystallized from hexane to give the title compound. Mp 144—145°C, yield 1 g (35%).

Example 15

N-methyl-4-piperidyl α-[N-(7-chloro-4-quinolyl)-anthraniloyloxy]-propionate (I, R=$CH_3$, $R_1$=$R_2$=$R_4$=H, $R_3$=$CH_3$, $R_5$=Cl).

A mixture of the sodium salt of N-(7-chloro-4-quinolyl)-anthranilic acid, 2.5 g of N-methyl-4-piperidyl α-bromopropionate and 20 ml of dimethylformamide was stirred and heated at 80°C for 5 hours. At the end of the reaction, after cooling, the inorganic salts were filtered off and the solvent evaporated off in vacuo. The oily residue was purified on alumina column using chloroform as eluent. The fractions containing the desired product were collected and the solvent evaporated off. Yield 4 g (85%).

The structure of the oily product was determined by NMR ($CDCl_3$)

| | | | |
|---|---|---|---|
| δ 10.4 | 1H | (s) | —NH |
| 8.6 | 1H | (d) | quinoline |
| 5.3 | 1H | (q) | O—CH—COO |
| 4.85 | 1H | (m) | piperidine |
| 2.15 | 3H | (s) | N—$CH_3$ |
| 1.62 | 3H | (d) | —O—CH(—$CH_3$)—COO |

**Claims for the Contracting States BE, CH, FR, GB, LI, SE:**

1. An N-substituted-4-(optionally substituted)-2-,3- or 4-piperidyl N-(7- or 8-substituted-4-quinolyl)-anthraniloyloxyalkanoate having the general formula I

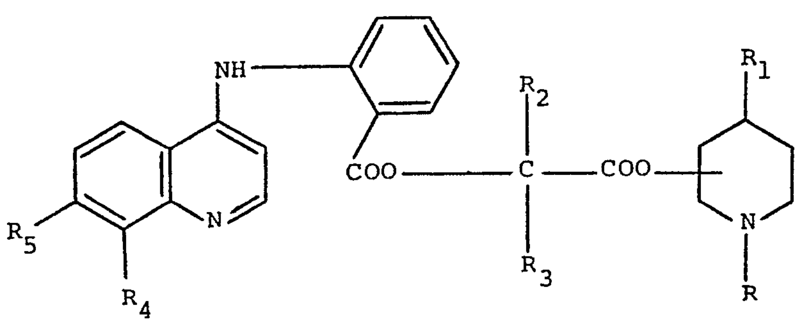
I

in which R represents a straight chain or branched chain alkyl group having from 1 to 4 carbon atoms; an alkenyl or alkynyl group having from 2 to 6 carbon atoms; a benzyl, phenethyl, 4-nitro-phenethyl or 4-amino-phenethyl group; or a phenacyl, benzoylethyl, β-hydroxy-phenethyl or α-hydroxy-phenylpropyl group, optionally substituted in the phenyl ring by one or more of a halogen atom, a trifluoromethyl, nitro or amino group, an alkyl group having from 1 to 4 carbon atoms or an alkoxy group having from 1 to 4 carbon atoms; $R_1$ represents a hydrogen atom or a phenyl group, each of $R_2$ and $R_3$ independently represents a hydrogen atom or an alkyl group having from 1 to 4 carbon atoms, one of $R_4$ and $R_5$ represents a chlorine atom or a trifluoromethyl group and the other of $R_4$ and $R_5$ represents a hydrogen atom; or a pharmaceutically acceptable salt of such an ester.

2. Any one of the following compounds according to claim 1:
N-methyl-3-piperidyl N-(7-chloro-4-quinolyl)-anthraniloyloxyacetate,
N-methyl-4-piperidyl N-(7-chloro-4-quinolyl)-anthraniloyloxyacetate,
N-methyl-4-phenyl-4-piperidyl N-(7-chloro-4-quinolyl)-anthraniloyloxyacetate,
N-ethyl-4-piperidyl N-(7-chloro-4-quinolyl)-anthraniloyloxyacetate,
N-butyl-4-piperidyl N-(7-chloro-4-quinoyl)-anthraniloyloxyacetate,
N-methyl-4-piperidyl N-(7-trifluoromethyl-4-quinolyl)-anthraniloyloxyacetate,
N-benzyl-4-piperidyl N-(7-chloro-4-quinolyl)-anthraniloyloxyacetate,
N-phenethyl-4-piperidyl N-(7-chloro-4-quinolyl)-anthraniloyloxyacetate,
N-phenacyl-4-piperidyl N-(7-chloro-4-quinolyl)-anthraniloyloxyacetate,
N-(*p*-nitrophenethyl)-4-piperidyl N-(7-chloro-4-quinolyl)-anthraniloyloxyacetate,
N-(*p*-amino-phenethyl)-4-piperidyl N-(7-chloro-4-quinolyl)-anthraniloyloxyacetate,
n-allyl-4-piperidyl N-(7-chloro-4-quinolyl)-anthraniloyloxyacetae,
N-propargyl-4-piperidyl N-(7-chloro-4-quinolyl)-anthraniloyloxyacetate,
N-benzyl-4-piperidyl N-(8-trifluoromethyl-4-quinolyl)-anthraniloyloxyacetate,
N-methyl-4-phenyl-4-piperidyl N-(8-trifluoromethyl-4-quinolyl)-anthraniloyloxyacetate,
N-methyl-4-piperidyl α-[N-(7-chloro-4-quinolyl)-anthraniloyloxy]-propionate.

3. A method for the preparation of an N-substituted-4-(optionally substituted)-2-,3- or 4-piperidyl N-(7- or 8-substituted-4-quinolyl)-anthraniloyloxyalkanoate according to claim 1, the method comprising reacting a substituted piperidine of the general formula II

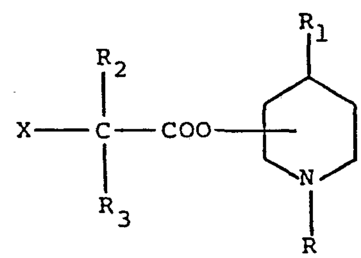

II

wherein X represents a halogen atom and R, $R_1$, $R_2$ and $R_3$ are as defined in claim 1 with an anthranilic acid derivative of the general formula III

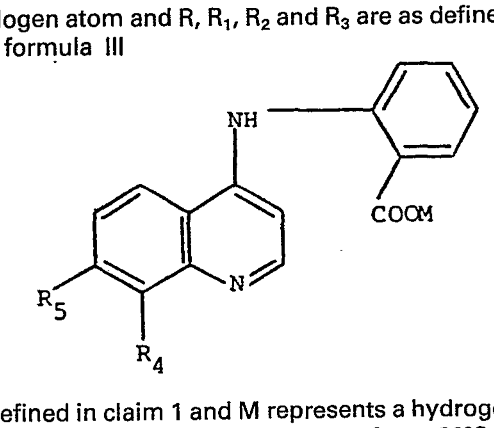

III

wherein $R_4$ and $R_5$ are as defined in claim 1 and M represents a hydrogen atom or an alkali metal atom, in a solvent in the presence of an alkali metal carbonate at from 20°C to 100°C.

4. A method according to claim 3 characterised in that the solvent is dimethylformamide.

5. A method according to claim 3 or claim 4 characterised in that the alkali metal carbonate is potassium carbonate.

6. A method according to any of claims 3 to 5 characterised in tha the reaction is carried out at from 50°C to 100°C.

7. A method according to any of claims 3 to 6 characterised in that the anthranilic acid derivative has the general formula III wherein M represents a sodium atom and $R_4$ and $R_5$ are as defined in claim 1.

8. A pharmaceutical composition comprising a compound according to claim 1 or claim 2 in admixture with a pharmaceutically acceptable diluent or carrier.

**Claims for the Contracting State AT:**

1. A method for the preparation of an N-substituted-4-(optionally substituted)-2-,3- or 4-piperidyl N-(7- or 8-substituted-4-quinoyl)-anthraniloylo1yalkanoate having the general formula I

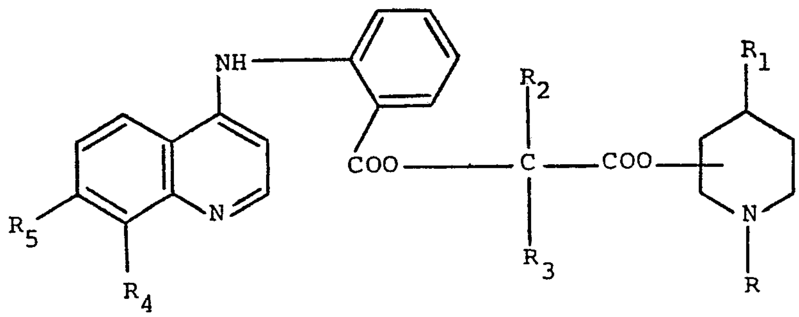

I

in which R represents a straight chain or branched chain alkyl group having from 1 to 4 carbon atoms; an alkenyl or alkynyl group having from 2 to 6 carbon atoms; a benzyl, phenethyl, 4-nitro-phenethyl or 4-amino-phenethyl group; or a phenacyl, benzoylethyl, β-hydroxy-phenethyl or α-hydroxy-phenylpropyl group, optionally substituted in the phenyl ring by one or more of a halogen atom, a trifluoromethyl, nitro or amino group, an alkyl group having from 1 to 4 carbon atoms or an alkoxy group having from 1 to 4 carbon atoms; $R_1$ represents a hydrogen atom or a phenyl group, each of $R_2$ and $R_3$ independently represents a hydrogen atom or an alkyl group having from 1 to 4 carbon atoms, one of $R_4$ and $R_5$ represents a chlorine atom or a trifluoromethyl group and the other of $R_4$ and $R_5$ represents a hydrogen atom; the method comprising reacting a substituted piperidine of the general formula II

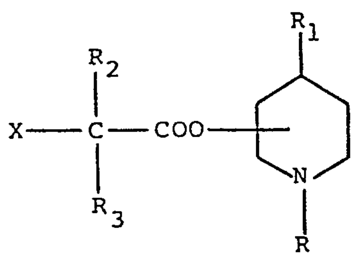

II

wherein x represents a halogen atom and R, $R_1$, $R_2$ and $R_3$ are as defined in this claim with an anthranilic acid derivative of the general formula III

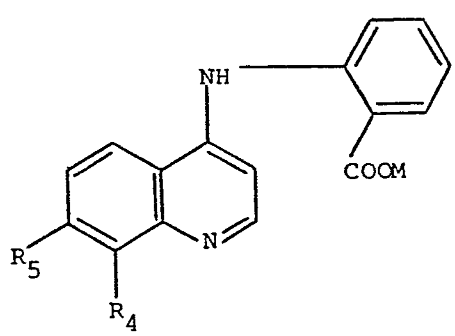

III

wherein $R_4$ and $R_5$ are as defined in this claim and M represents a hydrogen atom or an alkali metal atom, in a solvent in the presence of an alkali metal carbonate at from 20°C to 100°C.

2. A method according to claim 1 characterised in that the solvent is dimethylformamide.

3. A method according to claim 1 or claim 2 characterised in that the alkali metal carbonate is potassium carbonate.

4. A method according to any preceding claim characterised in that the reaction is carried out at from 50°C to 100°C.

5. A method according to any preceding claim characterised in that the anthranilic acid derivative has the general formula III wherein M represents a sodium atom and $R_4$ and $R_5$ are as defined in claim 1.

**Patentansprüche für die Vertragsstaaten BE, CH, FR, GB, LI, SE:**

1. Ein N-substituiertes-4-(gegebenenfalls substituiertes)-2-,3- oder 4-Piperidyl N-(7- oder 8-substituiertes-4-chinolyl)-anthraniloyloxyalkanoat der allgemeinen Formel I

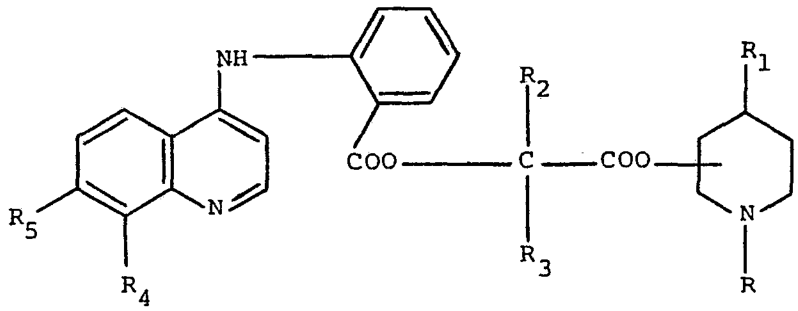

I

in der R für eine gerade oder verzweigte Alkylgruppenkette mit 1 bis 4 Kohlenstoffatomen; einer Alkenyl oder Alkynylgruppe mit 2 bis 6 Kohlenstoffatomen; einer Benzyl, Phenäthyl, 4-Nitro-phenäthyl oder 4-Amino-phenäthylgruppe; oder einer Phenacyl, Benzoyläthyl, β-Hydroxy-phenäthyl oder -Hydroxy-phenylpropylgruppe, gegebenenfalls im Phenylring mit einem oder mehreren Halogenatomen, einer Trifluoromethyl, Nitro- oder Aminogruppe, einer Alkylgruppe mit 1 bis 5 Kohlenstoffatomen oder einer Alkoxygruppe mit 1 bis 4 Kohlenstoffatomen steht; $R_1$ ein Wasserstoffatom oder eine Phenylgruppe und $R_2$ und $R_3$ jeweils unabhängig voneinander ein Wasserstoffatom oder eine Alkylgruppe mit 1 bis 4 Kohlenstoffatomen repräsentiert und eines von $R_4$ und $R_5$ ein Chloratom oder eine Trifluoromethylgruppe und das andere von $R_4$ und $R_5$ für ein Wasserstoffatom steht oder ein pharmazeutisch akzeptierbares Salz eines solchen Esters.

2. Eine der folgenden Verbindungen gemäß Anspruch 1:
N-Methyl-3-piperidyl N-(7-chloro-4-chinolyl)-anthraniloyloxyacetat,
N-Methyl-4-piperidyl N-(7-chloro-4-chinolyl)-anthraniloyloxyacetat,
N-Methyl-4-phenyl-4-piperidyl N-(7-chloro-4-chinolyl)-anthraniloyloxyacetat,
N-Äthyl-4-piperidyl N-(7-chloro-4-chinolyl)-anthraniloyloxyacetat,
N-Butyl-4-piperidyl N-(7-chloro-4-chinolyl)-anthraniloyloxyacetat,
N-Methyl-4-piperidyl N-(7-trifluoromethyl-4-chinolyl)-anthraniloyloxyacetat,
N-Benzyl-4-piperidyl N-(7-chloro-4-chinolyl)-anthraniloyloxyacetat,
N-Phenäthyl-4-piperidyl N-(7-chloro-4-chinolyl)-anthraniloyloxyacetat,
N-Phenacyl-4-piperidyl N-(7-chloro-4-chinolyl)-anthraniloyloxyacetat,
N-(*p*-Nitro-phenäthyl)-4-piperidyl N-(7-chloro-4-quinolyl)-anthraniloyloxyacetat,
N-(*p*-Amino-phenäthyl)-4-piperidyl N-(7-chloro-4-quinolyl)-anthraniloyloxyacetat,
N-Allyl-4-piperidyl N-(7-chloro-4-chinolyl)-anthraniloyloxyacetat,
N-Propargyl-4-piperidyl N-(7-chloro-4-chinolyl)-anthraniloyloxyacetat,
N-Benzyl-4-piperidyl N-(8-trifluoromethyl-4-chinolyl)-anthraniloyloxyacetat,
N-Methyl-4-phenyl-4-piperidyl N-(8-trifluoromethyl-4-quinolyl)-anthraniloyloxyacetat,
N-Methyl-4-piperidyl-[N-(7-chloro-4-chinolyl)-anthraniloyloxy]-propionat.

3. Verfahren zur Herstellung eines N-substituierten-4-(gegebenenfalls substituierten)-2-,3- oder 4-Piperidyl N-(7- oder 8-substituierten-4-chinolyl)-anthraniloyloxyalkanoat gemäß Anspruch 1, bei dem ein substituiertes Piperidin der allgemeinen Formel II

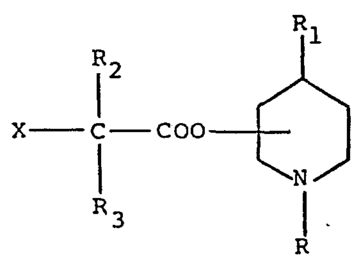

II

wobei X ein Halogenatom und R, $R_1$, $R_2$ und $R_3$ die in Anspruch 1 definierte Bedeutung haben, mit einem Anthranilsäurederivat der allgemeinen Formel III

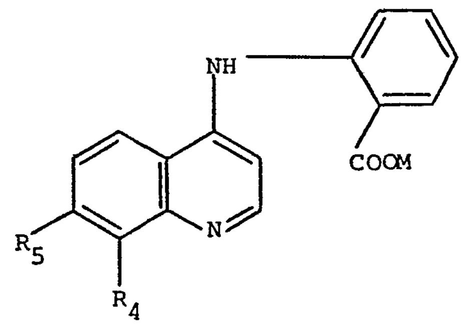

III

worin $R_4$ und $R_5$ die in Anspruch 1 definierte Bedeutung haben und M ein Wasserstoffatom oder ein Alkalimetallatom bedeutet, in einem Lösungsmittel in Gegenwart eines Alkalimetallcarbonates bei 20°C bis 100°C zur Reaktion gebracht wird.

4. Verfahren nach Anspruch 3, dadurch gekennzeichnet, daß das Lösungsmittel Dimethylformamid ist.

5. Verfahren nach Anspruch 3 oder 4, dadurch gekennzeichnet, daß das Alkalimetallcarbonat Kaliumcarbonat ist.

6. Verfahren nach einem der Ansprüche 3 bis 5, dadurch gekennzeichnet, daß die Reaktion der 50°C bis 100°C stattfindet.

7. Verfahren nach einem der Ansprüche 3 bis 6, dadurch gekennzeichnet, daß das Anthranilsäurederivat die allgemeine Formel III aufweist, wobei M ein Natriumatom ist, und $R_4$ und $R_5$ die im Anspruch 1 definierte Bedeutung haben.

8. Pharmazeutische Zusammensetzung mit einer Verbindung gemäß Anspruch 1 oder 2 in Mischung mit einem pharmazeutisch-annehmbaren Lösungsmittel oder Träger.

**Patentansprüche für den Vertragsstaat AT:**

1. Verfahren zur Herstellung eines N-substituierten-4-(gegebenenfalls substituierten(-2-,3- oder 4-Piperidyl N-(7- oder 8-substituierten-4-chinolyl)-anthraniloyloxyalkanoat der allgemeinen Formal I

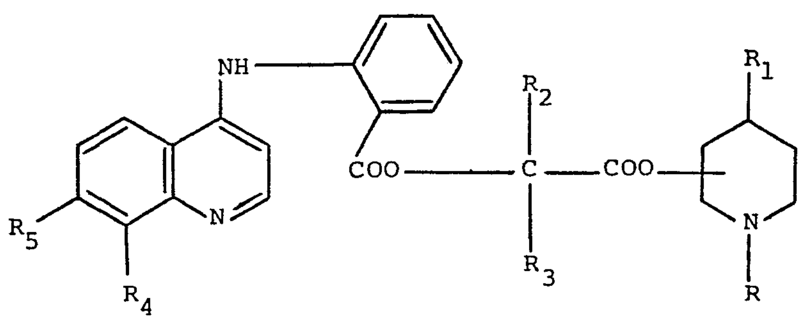

I

in der R für eine gerade oder verzweigte Alkylgruppenkette mit 1 bis 4 Kohlenstoffatomen; einer Alkenyl oder Alkynylgruppe mit 2 bis 6 Kohlenstoffatomen; einer Benzyl, Phenäthyl, 4-Nitro-Phenäthyl oder 4-Amino-phenäthylgruppe; oder einer Phenacyl, Benzoyläthyl, β-Hydroxy-phenäthyl oder -Hydroxy-phenylpropylgruppe, gegebenenfalls im Phenylring mit einem oder mehreren Halogenatomen, einer Trifluoromethyl, Nitro- oder Aminogruppe, einer Alkylgruppe mit 1 bis 5 Kohlenstoffatomen oder einer Alkoxygruppe mit 1 bis 4 Kohlenstoffatomen steht; $R_1$ ein Wasserstoffatom oder eine Phenylgruppe und $R_2$ und $R_3$ jeweils unabhängig voneinander ein Wasserstoffatom oder eine Alkylgruppe mit 1 bis 4 Kohlenstoffatomen repräsentiert und eines von $R_4$ und $R_5$ ein Chloratom oder eine Trifluoromethylgruppe und das andere von $R_4$ und $R_5$ für ein Wasserstoffatom steht oder ein pharmazeutisch akzeptierbares Salz eines solchen Esters; wobei das Verfahren die Reaktion eines substituierten Piperidins der allgemeinen Formel II

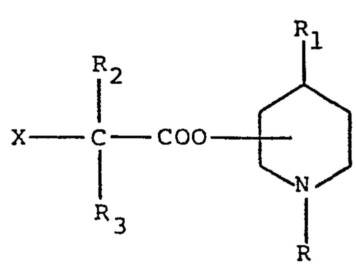

II

wobei X ein Halogenatom und R, $R_1$, $R_2$ und $R_3$ die oben definierte Bedeutung haben, mit einem Anthranilsäurederivat der allgemeinen Formel III

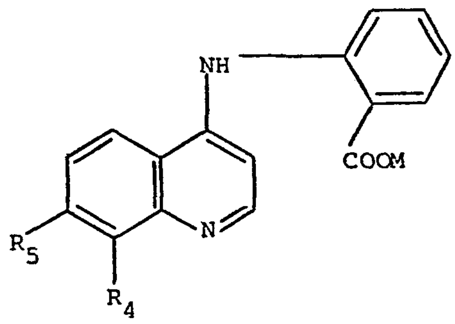

III

worin $R_4$ und $R_5$ die oben definierte Bedeutung haben und M ein Wasserstoffatom oder ein Alkalimetallatom sein kann, in einem Lösungsmittel in Gegenwart eines Alkalimetallcarbonates bei 20°C bis 100°C umfaßt.

2. Verfahren nach Anspruch 1, dadurch gekennzeichnet, daß das Lösungsmittel Dimethylformamid ist.

3. Verfahren nach Anspruch 1 oder 2, dadurch gekennzeichnet, daß das Alkalimetallcarbonat Kaliumcarbonat ist.

4. Verfahren nach einem der Ansprüche dadurch gekennzeichnet, daß die Reaktion der 50°C bis 100°C stattfindet.

5. Verfahren nach einem der Ansprüche, dadurch gekennzeichnet, daß das Anthranilsäurederivat die allgemeine Formel III aufweist, wobei M ein Natriumatom ist, und $R_4$ und $R_5$ die im Anspruch 1 definierte Bedeutung haben.

**Revendications pour les Etats Contractants BE, CH, FR, GB, LI, SE:**

1. Un N-(quinoléyl-4 substitué en position 7 ou 8) antraniloyloxyalcanoate, de pipéridine-yl 2 ou 3 ou 4 N-substitué et, éventuellement, 4-substitué répondant à la formule générale I

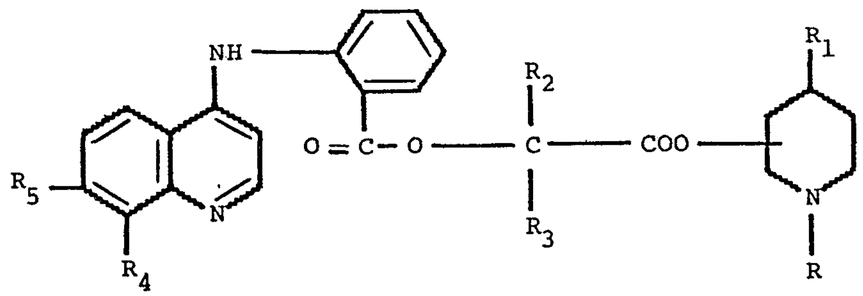

I

dans laquelle R représente un radical alcoyle en chaîne droite ou ramifiée comportant de 1 à 4 atomes de carbone, un radical alcènyle ou alcinyle ayant de 2 à 6 atomes de carbone, un groupement benzyle, phénéthyle, nitro-4 phénéthyle, amino-4 phénéthyle, phénacyle, benzoyléthyle, β-hydroxy phénéthyle ou α-hydroxy phénylpropyle éventuellement substitué sur le noyau phényle par un ou plusieurs atomes d'halogène, ou un radical trifluorométhyle ou un groupement nitro ou amino ou un radical alcoyle ayant de 1 à 4 atomes de carbone ou un radical alcoxy ayant de 1 à 4 atomes de carbone; $R_1$ représente un atome d'hydrogène ou un groupement phényle; chacun des radicaux $R_2$ ou $R_3$ représente, indépendamment, un atome d'hydrogène ou un radical alcoyle ayant de 1 à 4 atomes de carbone et $R_4$ et $R_5$ sont tels que l'un d'eux représente un atome de chlore ou un radical trifluorométhyle tandis que l'autre représente un atome d'hydrogène; ou un sel pharmaceutiquement acceptable de cet ester.

2. Un des composés selon la revendication 1:

N-(chloro-7 quinoléyl-4)antraniloyloxyacétate de N-méthyle-pipéridyle-3,
N-(chloro-7 quinoléyl-4)antraniloyloxyacétate de N-méthyle-pipéridyle-4,
N-(chloro-7 quinoléyl-4)antraniloyloxyacétate de N-méthyle-phényle-4-pipéridyle-4,
N-(chloro-7 quinoléyl-4)antraniloyloxyacétate de N-éthyle-pipéridyle-4,
N-(chloro-7 quinoléyl-4)antraniloyloxyacétate de N-butyl-pipéridyle-4,
N-(trifluorométhyle-7 quinoléyl-4)antraniloyloxyacétate de N-méthyle-pipéridyle-4,
N-(chloro-7 quinoléyl-4)antraniloyloxyacétate de N-benzyl-pipéridyle-4,
N-(chloro-7 quinoléyl-4)antraniloyloxyacétate de N-phénéthyle-pipéridyle-4,
N-(chloro-7 quinoléyl-4)antraniloyloxyacétate de N-phénacyle-pipéridyle-4,
N-(chloro-7 quinoléyl-4)antraniloyloxyacétate de N-(*p*-nitrophénéthyle)pipéridyle-4,
N-(chloro-7 quinoléyl-4)antraniloyloxyacétate de N-(*p*-aminophénéthyle)pipéridyle-4,

N-(chloro-7 quinoléyl-4)antraniloyloxyacétate de N-allyle-pipéridyle-4,
N-(chloro-7 quinoléyl-4)antraniloyloxyacétate de N-propargyle-pipéridyle-4,
N-(trifluorométhyle-8 quinoléyl-4)antraniloyloxyacétate de N-benzyl-pipéridyle-4,
N-(trifluorométhyle-8 quinoléyl-4)antraniloyloxyacétate de N-méthyl-phényle-4-pipéridyle-4,
N-(chloro-7 quinoléyl-4)antraniloyloxy (méthyle-2)acétate de N-méthyle-pipéridyle-4.

3. Une méthode pour la préparation d'un N-(quinoléyl-4 substitué en position 7 ou 8) antraniloyloxyalcanoate de pipéridine-yl 2 ou 3 ou 4 N-substitué et, éventuellement, 4-substitué selon la revendication 1, cette méthode comprenant l'étape de faire réagir une pipéridine substituée répondant à la formule générale II

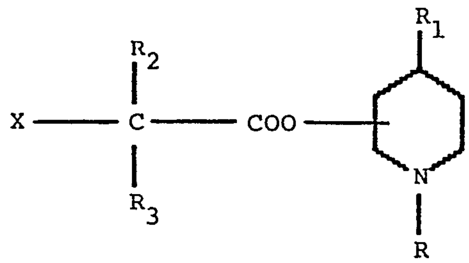

II

dans laquelle X représente un atome d'halogène et R, $R_1$, $R_2$ et $R_3$ sont comme définis dans la revendication 1, avec un dérivé de l'acide antranilique répondant à la formule générale III

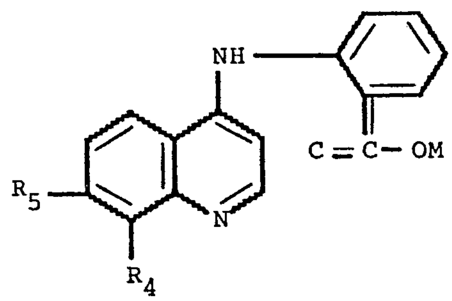

III

dans laquelle $R_4$ et $R_5$ sont définis comme dans la revendication 1 et M représente un atome d'hydrogène ou un atome de métal alcalin, dans un solvant, en présence d'un carbonate de métal alcalin et à une température comprise entre 20°C et 100°C.

4. Une méthode selon la revendication 3, caractérisé en ce que le solvant est le diméthylformamide.

5. Une méthode selon l'une des revendications 3 ou 4, caractérisée en ce que le carbonate de métal alcalin est le carbonate de potassium.

6. Une méthode selon l'une des revendications 3 à 5, caractérisée en ce que la réaction est effectuée à une température comprise entre 50°C et 100°C.

7. Une méthode selon l'une des revendications 3 à 6, caractérisée en ce que le dérivé de l'acide antranilique répond à la formule générale III dans laquelle M représente un atome de sodium et $R_4$ et $R_5$ sont comme définis dans la revendication 1.

8. Une composition pharmaceutique comprenant un composé selon l'une des revendications 1 ou 2, mélangé avec un diluant ou un excipient pharmaceutiquement acceptable.

**Revendications pour l'Etat Contractant AT:**

1. Une méthode pour la préparation d'un N-(quinoléyl-4 substitué en position 7 ou 8) antraniloyloxyalcanoate de pipéridine-yl 2 ou 3 ou 4 N-substitué et, éventuellement, 4-substitué répondant à la formule générale I

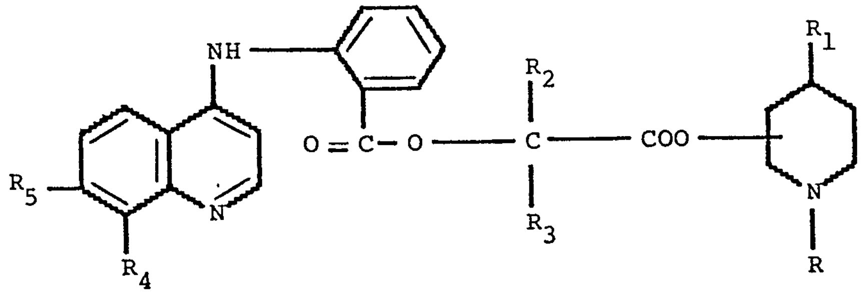

I

dans laquelle R représente un radical alcoyle en chaîne droite ou ramifiée comportant de 1 à 4 atomes de carbone, un radical alcènyle ou alcinyle ayant de 2 à 6 atomes de carbone, un groupement benzyle, phénéthyle, nitro-4 phénéthyle, amino-4 phénéthyle, phénacyle, benzoyléthyle, β-hydroxy phénéthyle ou α-hydroxy phénylpropyle éventuellement substitué sur le noyau phényle par un ou plusieurs atomes d'halogène, ou un radical trifluorométhyle ou un groupement nitro ou amino ou un radical alcoyle ayant de 1 à 4 atomes de carbone ou un radical alcoxy ayant de 1 à 4 atomes de carbone; $R_1$ représente un atome d'hydrogène ou un groupement phényle; chacun des radicaux $R_2$ ou $R_3$ représente, indépendamment, un atome d'hydrogène ou un radical alcoyle ayant de 1 à 4 atomes de carbone et $R_4$ et $R_5$ sont tels que l'un d'eux représente un atome de chlore ou un radical trifluorométhyle tandis que l'autre représente un atome d'hydrogène, cette méthode comprenant l'étape de faire réagir une pipéridine substituée répondant à la formule générale II

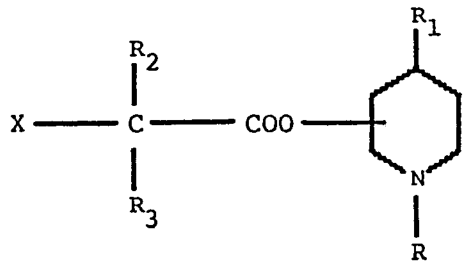

II

dans laquelle X représente un atome d'halogène et R, $R_1$, $R_2$ et $R_3$ sont comme définis dans cette revendication, avec un dérivé de l'acide antranilique répondant à la formule générale III

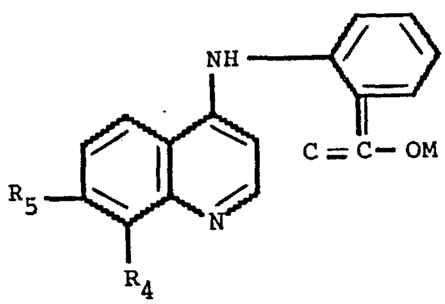

III

dans laquelle $R_4$ et $R_5$ sont définis comme dans cette revendication et M représente un atome d'hydrogène ou un atome de métal alcalin, dans un solvant, en présence d'un carbonate de métal alcalin et à une température comprise entre 20°C et 100°C.

2. Une méthode selon la revendication 1, charactérisée en ce que le solvant est le diméthylformamide.

3. Une méthode selon l'une des revendications 1 ou 2, caractérisée en ce que le carbonate de métal alcalin est le carbonate de potassium.

4. Une méthode selon l'une des revendications précédentes, caractérisée en ce que la réaction est effectuée à une température comprise entre 50°C et 100°C.

5. Une méthode selon l'une des revendications précédentes, caractérisée en ce que le dérivé de l'acide antranilique répond à la formule générale III dans laquelle M représente un atome de sodium et $R_4$ et $R_5$ sont comme définis dans la revendication 1.